# EUROPÄISCHE PATENTSCHRIFT

(11) **EP 2 274 268 B1**
(45) Veröffentlichungstag und Bekanntmachung des Hinweises auf die Patenterteilung: **14.12.2011**
(21) Anmeldenummer: 09729611.5
(22) Anmeldetag: 07.04.2009
(51) Int. Cl.: C07C 209/60, B01J 37/00

(54) **VERFAHREN ZUR VORBEHANDLUNG VON HYDROAMINIERUNGSKATALYSATOREN**
METHOD FOR PRE-TREATING HYDROAMINATION CATALYSTS
PROCÉDÉ DE PRÉTRAITEMENT DE CATALYSEURS D'HYDROAMINATION

(30) Priorität: 09.04.2008 EP 08154266
(43) Veröffentlichungstag der Anmeldung: 19.01.2011
(73) Patentinhaber: BASF SE, 67056 Ludwigshafen (DE)
(72) Erfinder: HEIDEMANN, Thomas, 68519 Viernheim (DE); SIGL, Marcus, 68165 Mannheim (DE)
(86) Internationale Anmeldenummer: PCT/EP2009/054124
(87) Internationale Veröffentlichungsnummer: WO 2009/124924

(56) Entgegenhaltungen:
- EP-A- 0 133 938
- DE-A1- 10 313 853
- DE-A1-102005 051 044

## Beschreibung

Die vorliegende Erfindung betrifft ein Verfahren zur Vorbehandlung von Hydroaminierungskatalysatoren. Ferner betrifft die Erfindung ein Verfahren zur Herstellung von Alkylaminen durch Umsetzung von Olefinen mit Ammoniak, primären oder sekundären Aminen an einem Hydroaminierungskatalysator, der vor der Umsetzung der Olefine mit Ammoniak mit einem Ammoniak-haltigen Gemisch in Kontakt gebracht wird, wobei das Ammoniak-haltige Gemisch weniger als 40 Gew.-% Olefin enthält.

Aus EP-A-0133938 ist bekannt, dass in der Hydroaminierungsreaktion eingesetzte Alumino-Zeolith-Katalysatoren schnell deaktivieren, da diese Katalysatoren eine Polymerisation bzw. Oligomerisation der eingesetzten Olefine auf dem Katalysator und die nachfolgende Verkokung dieser polymeren bzw. oligomeren Rückstände auf den Katalysatoren begünstigen. EP-A-0133938 lehrt, dass die Deaktivierung der Katalysatoren verringert werden kann, wenn man Ammoniak, primäre oder sekundäre Amine mit Olefinen in Gegenwart von Borosilikat- oder Borogermanatzeolith-Katalysatoren des Pentasilstyp umsetzt, das erhaltene Amin abtrennt und die nicht umgesetzten Einsatzstoffe zurückführt. Weiterhin wird offenbart, dass man deaktivierte Katalysatoren regenerieren kann, in dem man Luft oder ein Luft/Stickstoff-Gemisch bei 400 bis 550°C über den Katalysator leitet.

WO-A-97/07088 offenbart die Hydroaminierung von Olefinen in Gegenwart von Bor-BETA-Zeolith-Katalysatoren. In dieser Anmeldung werden verschiedene Faktoren genannt, die die Deaktivierung von Katalysatoren beeinflussen können. So wird gelehrt, dass Monoolefine eine geringer ausgeprägte Polymerisationsneigung aufweisen als Dioder Polyolefine. Höhere Temperaturen sollen die Polymerisation und die Crack-Reaktion der eingesetzten Olefine fördern. Eine Wiederherstellung der Aktivität der Katalysatoren kann offenbarungsgemäß durch Regenerierung in einem sauerstoffhaltigen Gas bei höheren Temperaturen erfolgen.

Der Stand der Technik lehrt, dass die Polymerisation der in die Hydroaminierungsreaktion eingesetzten Olefine und die daraus resultierende Verkokung zu einer Deaktivierung der eingesetzten Katalysatoren führen können. Faktoren, wie die Art und Beschaffenheit des Katalysators, die Reaktionstemperatur, die Art des Olefins können die Geschwindigkeit und den Grad der Deaktivierung beeinflussen. Üblicherweise wird die Aktivität durch eine Regenerierung der Katalysatoren wiederhergestellt, in dem man ein sauerstoffhaltiges Gas bei einer Temperatur im Bereich von 400 bis 550°C über den Katalysator leitet.

DE-A-10313853 lehrt, dass die Aktivität von calcinierten, zeolithischen Katalysatoren gesteigert werden kann, wenn der calcinierte Katalysator maximal 24 Stunden vor Beginn der Umsetzung bei Temperaturen im Bereich von 100 bis 550°C in einem sauerstoffhaltigen Gasstrom behandelt wird. Offenbarungsgemäß tritt eine Erhöhung der Aktivität unabhängig davon auf, ob der Katalysator erstmalig in die Hydroaminierung eingesetzt wird oder ob ein bereits regenerierter Katalysator eingesetzt wird.

Aus DE-A-102005051044 kann entnommen werden, dass die Hydroaminierung in der Regel in einer adiabat betriebenen Reaktionseinheit erfolgt.

Beim Anfahren von zeolithischen Katalysatoren, d.h. dem ersten Inkontaktbringen des Katalysators mit dem Eduktgemisch aus Olefin und Ammoniak bzw. Amin, wird in der Regel ein hoher Temperaturanstieg beobachtet, da die beim Anfahren produzierte Wärme gerade bei großtechnischen, adiabat betriebenen Reaktoren nur sehr schlecht abgeführt werden kann. Diese Temperaturerhöhung begünstigt in der Regel die Oligomerisierung der eingesetzten Olefine. Da die Oligomerisierung im Allgemeinen eine exotherme Reaktion ist, beschleunigt die bei dieser Reaktion frei werdende Wärme den Temperaturanstieg zusätzlich. Die bei dem Anfahr-Prozess auftretenden hohen Temperaturen führen in der Regel zu einer Schädigung des Katalysators durch Ablagerung der Olefin-Oligomere auf der Katalysatoroberfläche und in den Poren oder gar zu einer Veränderung an der Zeolithstruktur selbst.

Es war demnach Ziel der vorliegenden Erfindung ein Hydroaminierungsverfahren zur Verfügung zu stellen, bei dem die thermische Schädigung der Hydroaminierungskatalysatoren beim Anfahren des Katalysators verringert wird. Es sollte ein Hydroaminierungsverfahren mit hohen Standzeiten bereitgestellt werden, das es ermöglicht Hydroaminierungsprodukt in einer hohen Ausbeute und Selektivität zu erhalten. Die Betriebsdauer des Katalysators bis zu einer ggf. erforderlichen Regenerierung sollte erhöht werden. Eine weitere Aufgabe der vorliegenden Erfindung bestand darin, ein Verfahren zur Vorbehandlung eines Hydroaminierungskatalysators zu entwickeln, dass zu einer geringeren thermischen Schädigung der eingesetzten Katalysatoren führt, so dass die Aktivität der Katalysatoren möglichst lange erhalten bleibt.

Erfindungsgemäß wurde die Aufgabe durch ein Verfahren zur Vorbehandlung von Hydroaminierungskatalysatoren, dadurch gekennzeichnet, dass man den Hydroaminierungskatalysator vor der Umsetzung von Olefinen mit Ammoniak, einem primären oder sekundären Amin mit einem Ammoniak-haltigen Gemisch in Kontakt bringt, wobei das Ammoniak-haltige Gemisch weniger als 40 Gew.-% Olefin enthält, gelöst.

Die Aufgabe wird ferner durch ein Verfahren zur Herstellung von Alkylaminen durch Umsetzung von Olefinen mit Ammoniak, primären oder sekundären Aminen an einem Hydroaminierungskatalysator, dadurch gekennzeichnet, dass man vor der Umsetzung der Olefine mit Ammoniak, primären oder sekundären Aminen den Hydroaminierungskatalysator mit einem Ammoniak-haltigen Gemisch in Kontakt bringt, wobei das Ammoniak-haltige Gemisch weniger als 40 Gew.-% Olefin enthält, gelöst.

Erfindungsgemäß werden Hydroaminierungskatalysatoren vor der Umsetzung von Olefinen mit Ammoniak, einem primären oder sekundären Amin mit einem Ammoniak-haltigen Gemisch in Kontakt gebracht, wobei das Ammoniak-haltige Gemisch weniger als 40 Gew.-% Olefin enthält.

Bevorzugt besteht das Ammoniak-haltige Gemisch aus handelsüblichem Ammoniak. In der Regel wird handelsüblicher Ammoniak mit einem Ammoniakgehalt von mehr als 95 Gew.-%, bevorzugt mehr als 98 Gew.-%, besonders bevorzugt mehr als 99 Gew.-%, insbesondere mehr als 99,5 Gew.-%, eingesetzt.

In einer weiteren bevorzugten Ausführungsform werden die Hydroaminierungskatalysatoren mit einem Ammoniak-haltigen Gemisch in Kontakt gebracht, das neben Ammoniak noch Olefin enthält. Bevorzugt ist das Olefin, das neben Ammoniak eingesetzt wird, das in die nachfolgende Hydroaminierungsreaktion eingesetzte Olefin. Erfindungsgemäß enthält das Ammoniak-haltige Gemisch weniger als 40 Gew.-% Olefin. Besonders bevorzugt enthält das Ammoniak-haltige Gemisch weniger als 10 Gew.-%, besonders bevorzugt weniger als 5 Gew.-%, insbesondere bevorzugt weniger als 1 Gew.-% Olefin.

In der Regel enthält das Ammoniak-haltige Gemisch neben Ammoniak und ggf. einem Olefin keine weiteren Bestandteile. Es ist aber möglich, dass das Ammoniak-haltige Gemisch noch weitere Bestandteile enthält, beispielsweise Wasser oder ein anderes primäres oder sekundäres Amin. In der Regel beträgt der Anteil an weiteren Bestandteile jedoch bevorzugt weniger als 5 Gew.-%, besonders bevorzugt weniger als 2 Gew.-% und insbesondere bevorzugt weniger als 1 Gew.-%.

Bevorzugt wird der Hydroaminierungskatalysator mit dem Ammoniak-haltigen Gemisch bei einer Temperatur von 0 bis 50°C, bevorzugt 10 bis 30°C, in Kontakt gebracht.

Der bevorzugte Druck, bei dem man den Hydroaminierungskatalysator mit dem Ammoniak-haltigen Gemisch in Kontakt bringt, liegt im Bereich von 0,1 bis 50 bar abs., bevorzugt im Bereich von 0,5 bis 20 bar abs. und besonders bevorzugt im Bereich von 1 bis 10 bar abs.

Das Ammoniak-haltige Gemisch kann gasförmig oder in flüssiger Form mit dem Hydroaminierungskatalysator in Kontakt gebracht werden.

Der Hydroaminierungskatalysator wird üblicherweise mit dem Ammoniak-haltigen Gemisch in Kontakt gebracht, in dem man den Reaktor, der den Katalysator enthält, mit flüssigem Ammoniak-haltigen Gemisch füllt. Bevorzugt wird sämtlicher im Reaktor befindlicher Hydroaminierungskatalysator mit flüssigem Ammoniak-haltigen Gemisch benetzt.

Das flüssige Ammoniak-haltige Gemisch kann auch kontinuierlich über den Hydroaminierungskatalysator geleitet werden. Die Katalysatorbelastung liegt im Allgemeinen im Bereich von (0,1 bis 20 kg Ammoniak-haltiges Gemisch)/( kg Katalysator)/Minute, bevorzugt (0,5 bis 10 kg Ammoniak-haltiges Gemisch)/( kg Katalysator)/Minute.

Das Ammoniak-haltige Gemisch kann auch gasförmig über den Hydroaminierungskatalysator geleitet werden. Die Katalysatorbelastung beträgt im Allgemeinen zwischen (0,1 bis 20 l Ammoniak-haltiges Gemisch)/( kg Katalysator)/Minute, bevorzugt (0,5 bis 10 l Ammoniak-haltiges Gemisch)/( kg Katalysator)/Minute.

Die Dauer des Inkontaktbringen des Hydroaminierungskatalysators mit dem Ammoniak-haltigen Gemisch liegt im Allgemeinen zwischen 5 Minuten und 24 Stunden, bevorzugt 10 Minuten bis 12 Stunden und besonders bevorzugt 30 Minuten bis 6 Stunden.

Der Hydroaminierungskatalysator kann außerhalb des Reaktors, in dem die Hydroaminierung durchgeführt wird, mit dem Ammoniak-haltigen Gemisch in Kontakt gebracht werden.

Bevorzugt wird der Hydroaminierungskatalysator jedoch in dem Reaktor mit dem Ammoniak-haltigen Gemisch in Kontakt gebracht, in dem die nachfolgende Hydroaminierung erfolgt. Dieser Reaktor ist bevorzugt ein kontinuierlich betriebener Reaktor, beispielsweise ein Rohr- oder ein Wirbelbettreaktor.

In einer bevorzugten Ausführungsform wird das Ammoniak-haltige Gemisch zusammen mit Inertgasen, beispielsweise Stickstoff, Argon, Helium oder Gemischen davon, mit dem Hydroaminierungskatalysator in Kontakt gebracht. Die Zuführung von Inertgasen zu dem Ammoniak-haltigen Gemisch hat den Vorteil, dass die Inertgase Wärme vom Katalysator ableiten können.

Der Fluss der Inertgase über den Hydroaminierungskatalysator liegt üblicherweise im Bereich von (1 l Inertgas)/(kg Katalysator)/Minute bis (1000 l Inertgas)/(kg Katalysator)/Minute, bevorzugt (5 l Inertgas)/(kg Katalysator)/Minute bis (100 l Inertgas)/(kg Katalysator)/Minute, besonders bevorzugt (10 l Inertgas)/(kg Katalysator)/Minute bis (50 l Inertgas)/(kg Katalysator)/Minute.

In einer besonders bevorzugten Ausführungsform wird der Hydroaminierungskatalysator gemäß der Lehre von DE-A-10313853 thermisch behandelt, bevor man ihn in Kontakt mit dem Ammoniak-haltigen Gemisch bringt. Die thermische Behandlung kann bevorzugt bei Temperaturen im Bereich von 100 bis 550°C in einem Gasstrom aus Luft, Stickstoff, anderen Inertgasen oder Gemischen davon erfolgen.

In einer bevorzugten Ausführungsform sind die im erfindungsgemäßen Verfahren eingesetzten Hydroaminierungskatalysatoren noch nicht in einer Hydroaminierungsreaktion eingesetzt worden, also solche Katalysatoren, an denen noch keine Umsetzung von Olefinen mit Ammoniak, primären oder sekundären Aminen erfolgt ist.

Es können aber auch Hydroaminierungskatalysatoren in das Verfahren eingesetzt werden, die bereits in einer Hydroaminierungsreaktion eingesetzt wurden und die im Anschluss an die Hydroaminierungsreaktion in einem sauerstoffhaltigen Gas bei erhöhten Temperaturen, vorzugsweise bei Temperaturen von 400 bis 550°C, in üblicher Weise regeneriert wurden.

Nach dem man den Hydroaminierungskatalysator mit dem Ammoniak-haltigen Gemisch in Kontakt gebracht hat, können an dem Hydroaminierungskatalysator Olefine mit Ammoniak, primären oder sekundären Aminen zu Alkylaminen umgesetzt werden. Die vorliegende Erfindung betrifft demgemäß ein Verfahren zur Herstellung von Alkylaminen durch Umsetzung von Olefinen mit Ammoniak, primären oder sekundären Aminen an einem Hydroaminierungskatalysator, dadurch gekennzeichnet, dass man vor der Umsetzung der Olefine mit Ammoniak, primären oder sekundären Aminen den Hydroaminierungskatalysator erfindungsgemäß vorbehandelt, in dem man den Hydroaminierungskatalysator mit einem Ammoniak-haltigen Gemisch in Kontakt bringt, wobei das Ammoniak-haltige Gemisch weniger als 40 Gew.-% Olefin enthält.

Als Hydroaminierungskatalysatoren werden bevorzugt calcinierte zeolithische Katalysatoren eingesetzt.

Bei zeolithischen Hydroaminierungskatalysatoren ist die Aktivmasse aus Zeolithen aufgebaut. Üblicherweise enthalten zeolithische Hydroaminierungskatalysatoren noch Bindemittel, die zur Herstellung von Katalysatorformkörpern erforderlich sind. Bei der Herstellung der Katalysatorformkörper aus entsprechenden Formmassen wird üblicherweise nach einer Trocknung noch calciniert, um zum letztendlichen Katalysator zu gelangen.

Der die Verformung der Katalysatorformkörper abschließende Schritt ist die Calcinierung. Hierbei wird in der Regel eine Temperatur von mehr als 400°C benötigt, damit das Bindermaterial aushärtet. Die Maximaltemperatur ist beschränkt durch die Stabilität des Zeolithen, der bei Temperaturen von mehr als 550°C seine Kristallinität verliert. Die Calcinierung wird großtechnisch im Drehrohr durchgeführt bei einer Temperatur im Bereich von 400 bis 560°C, einer Verweilzeit von 2 bis 4 Stunden. Im Labor wird üblicherweise in einem Ofen gearbeitet bei einer Temperatur von 480 bis 520°C und einem Zeitraum von 2 bis 32 Stunden.

Hydroaminierungskatalysatoren für die Hydroaminierung von Olefinen mit Ammoniak und/oder einem primären bzw. sekundären Amin sind in der Regel Zeolithe, insbesondere Faujasite wie X-, Y- und USY-Zeolith, Erionit, Chabazit, Mordenit, Offretit, Clinoptiolith, Pentasile wie ZSM-5 und ZBM-10, ZSM-11, ZSM-12, MCM-22, MCM-41, MCM-48, MCM-49, MCM-56, EMT, SSZ-26, SSZ-33, SSZ-37, CIT-1, PSH-3, NU-85, Beta sowie die borhaltigen Formen, wie zum Beispiel ZBM-11, H-Bor-ZSM-5, H-Bor-Beta, H-Bor-ZSM-11 sowie die gallium- oder titanhaltigen Formen. Sie zeichnen sich durch eine hohe Anzahl an katalytisch aktiven Zentren aus, kombiniert mit einer großen Oberfläche.

Die beschriebenen Zeolithe unterscheiden sich im Typ und in der Art der Nachbehandlung nach ihrer Herstellung (zum Beispiel thermische Behandlung, Dealuminierung, Säurebehandlung, Metallioneneintausch, etc.).

Beispiele für Zeolithe finden sich in US-A 4,375,002, US-A 4,536,602, EP-A 305 564, EP-A 101 921 und DE-A 42 06 992.

Auch die aus EP-A 133 938, EP-A 431 451 und EP-A 132 736 bekannten Zeolithe, bei denen es sich um Bor-, Gallium-, Alumino- und Eisensilikatzeolithe handelt, die gegebenenfalls wie beschrieben mit Alkali-, Erdalkali- und Übergangsmetallen dotiert werden können, können in das erfindungsgemäße Verfahren eingesetzt werden.

Weiterhin können zum Beispiel auch die aus CA-A 2 092 964 bekannten Beta-Zeolithe, die als kristalline Aluminosilikate bestimmter Zusammensetzung mit einer Porengröße von mehr als 5 Å definiert sind, verwendet werden.

Bevorzugt werden metall- oder halogenmodifizierte Beta-Zeolithe eingesetzt, wie zum Beispiel in DE-A 195 30 177 beschrieben.

Insbesondere bevorzugt sind auch Zeolith-Katalysatoren des Pentasil-Typs mit einem SiO₂/Al₂O₃-Molverhältnis von größer/gleich 10, wie sie in EP-A 132 736 offenbart sind.

Unter die Aluminiumphosphate und Silico-Alumophosphate fallen die kristallinen Systeme mit Zeolithstrukturen oder zeolithähnlichen Strukturen wie zum Beispiel SAPO-37, AlPO₄-5, SAPO-5, wie in DE-A 196 01 409 beschrieben, aber auch amorphe Systeme, wie sie zum Beispiel in DE-A 44 31 093 beschrieben sind. Sie besitzen allgemein die Formel Al₂O₃*P₂O₅*xSiO₂.

Die Hydroaminierungskatalysatoren können in Form von Pulver oder bevorzugt in Form von Formkörpern wie Strängen, Tabletten oder Splitt eingesetzt werden. Für die Verformung können 2 bis 60 Gew.-% (bezogen auf die zu verformende Masse) Bindemittel zugesetzt werden. Als Bindemittel eignen sich verschiedene Aluminiumoxide, bevorzugt Boehmit, amorphe Aluminosilikate mit einem molaren SiO₂/Al₂O₃-Verhältnis von 25:75 bis 95:5, Siliciumdioxid, bevorzugt hochdisperses SiO₂ wie zum Beispiel Silikasole, Gemische aus hochdispersem SiO₂ und hochdispersem Al₂O₃, hochdisperses TiO₂ sowie Tone.

In der Regel werden die Hydroaminierungskatalysatoren in der H-Form eingesetzt. Zur Erhöhung der Selektivität, der Standzeit und der Anzahl der möglichen Katalysatorregenerierungen kann man jedoch zudem verschiedene Modifizierungen an den Hydroaminierungskatalysatoren vornehmen.

Eine Modifizierung der Hydroaminierungskatalysatoren besteht darin, das man die unverformten Hydroaminierungskatalysatoren mit Alkalimetallen wie Na und K, Erdalkalimetallen wie Ca, Mg, Schwermetallen wie TI, Übergangsmetallen wie beispielsweise Mn, Fe, Mo, Cu, Zn, Cr, Edelmetallen und/oder seltenen Erdmetallen wie zum Beispiel La, Ce oder Y Ionenaustauschen bzw. dotieren kann.

Eine vorteilhafte Katalysatorausführungsform besteht darin, dass man die verformten Hydroaminierungskatalysatoren in einem Strömungsrohr vorlegt und bei 20 bis 100°C zum Beispiel ein Halogenid, ein Acetat, ein Oxalat, ein Citrat oder ein Nitrat der oben beschriebenen Metalle in gelöster Form darüberleitet. Ein derartiger Ionenaustausch kann zum Beispiel an der Wasserstoff-, Ammonium- und Alkaliform der Hydroaminierungskatalysatoren vorgenommen werden.

Eine andere Möglichkeit der Metallaufbringung auf die Hydroaminierungskatalysatoren besteht darin, dass man das zeolithische Material zum Beispiel mit einem Halogenid, Acetat, Oxalat, Citrat, Nitrat oder Oxid der oben beschriebenen Metalle in wässriger oder alkoholischer Lösung imprägniert.

Sowohl an einen Ionenaustausch als auch an eine Imprägnierung kann man eine Trocknung, wahlweise eine abermalige Calcination anschließen. Bei den metalldotierten Hydroaminierungskatalysatoren kann eine Nachbehandlung mit Wasserstoff und/oder mit Wasserdampf günstig sein.

Eine weitere Möglichkeit der Modifizierung des Katalysators besteht darin, dass man das heterogenkatalytische Material - verformt oder unverformt - einer Behandlung mit Säuren, wie Salzsäure (HCl), Flusssäure (HF), Phosphorsäure (H₃PO₄), Schwefelsäure (H₂SO₄), Oxalsäure (HO₂C-CO₂H) oder deren Gemischen unterwirft.

Eine besondere Ausführungsform besteht darin, dass man das Katalysatorpulver vor seiner Verformung mit Flusssäure (0,001 bis 2 molar, bevorzugt 0,05 bis 0,5 molar) 1 bis 3 Stunden unter Rückfluss behandelt. Nach Abfiltrieren und Auswaschen wird in der Regel bei 100 bis 160°C getrocknet und bei 400 bis 550°C calciniert.

Eine weitere besondere Ausführungsform liegt in einer HCI-Behandlung der Heterogenkatalysatoren nach ihrer Verformung mit Bindemittel. Hierbei wird der Heterogenkatalysator in der Regel 1 bis 3 Stunden bei Temperaturen zwischen 60 und 80°C mit einer 3 bis 25%igen, insbesondere mit einer 12 bis 20%igen Salzsäure behandelt, anschließend ausgewaschen, bei 100 bis 160°C getrocknet und bei 400 bis 550°C calciniert.

Eine andere Möglichkeit der Modifizierung des Katalysators ist der Austausch mit Ammoniumsalzen, zum Beispiel mit NH₄Cl, oder mit Mono-, Di- oder Polyaminen. Hierbei wird der mit Bindemittel verformte Heterogenkatalysator in der Regel bei 60 bis 80°C mit 10 bis 25%iger, bevorzugt ca. 20%iger NH₄Cl-Lösung 2 Stunden kontinuierlich in gewichtsmäßiger Heterogenkatalysator/Ammoniumchlorid-Lösung von 1:15 ausgetauscht und danach bei 100 bis 120°C getrocknet.

Eine weitere Modifikation, die an aluminiumhaltigen Hydroaminierungskatalysatoren vorgenommen werden kann, ist eine Dealuminierung, bei der ein Teil der Aluminiumatome durch Silicium ersetzt wird oder die Hydroaminierungskatalysatoren durch beispielsweise hydrothermale Behandlung in ihrem Aluminiumgehalt abgereichert werden. An eine hydrothermale Dealuminierung schließt sich vorteilhafterweise eine Extraktion mit Säuren oder Komplexbildnern an, um gebildetes Nichtgitteraluminium zu entfernen. Der Ersatz von Aluminium durch Silicium kann beispielsweise mit Hilfe von (NH₄)₂SiF₆ oder SiCl₄ erfolgen. Beispiele für Dealuminierungen von Y-Zeolithen finden sich in Corma et al., Stud. Surf. Sci. Catal. 37 (1987), Seiten 495 bis 503.

Die Hydroaminierungskatalysatoren kann man als Stränge mit Durchmessern von zum Beispiel 1 bis 4 mm oder als Tabletten mit zum Beispiel 3 bis 5 mm Durchmesser für die Hydroaminierung der Olefine einsetzen.

Nach der Verformung werden die Extrudate oder Presslinge zweckmäßig bei 80 bis 150°C für 2 bis 16 Stunden, zum Beispiel bei 110°C/16 Stunden getrocknet und bei 300 bis 500°C/ 2 bis 16 Stunden calciniert, wobei die Calcinierung wie die Aktivierung auch direkt im Hydroaminierungsreaktor erfolgen kann.

In einer bevorzugten Ausführungsform werden calcinierte Hydroaminierungskatalysatoren in das erfindungsgemäße Verfahren eingesetzt.

Die Umsetzung des Olefins mit Ammoniak und/oder dem primären oder sekundärem Amin in Gegenwart der anorganischen Festkörpersäure kann zum Beispiel wie in EP-A 132 736, EP-A 752 409, EP-A 822 179 und WO-A-02/00597 beschrieben erfolgen.

Die Reaktion kann kontinuierlich, in Batch-Fahrweise oder in Semi-Batch-Fahrweise betrieben werden.

Bevorzugt geht man dabei in der Regel so vor, dass man Ammoniak und/oder primäres Amin oder gegebenenfalls sekundäres Amin zusammen mit Olefin in einem molaren Verhältnis von 1:1 bis 10:1, bevorzugt 1:1 bis 5:1, insbesondere bevorzugt 1:1 bis 3:1, mischt und in einem Festbett- oder in einer Wirbelschichtreaktor, der den erfindungsgemäß vorbehandelten Hydroaminierungskatalysator enthält, bei einem Druck von 40 bis 700 bar abs., bevorzugt 200 bis 300 bar abs., und einer Temperatur von 80 bis 400°C, bevorzugt 230 bis 320°C, in der Gasphase oder im überkritischen Zustand umsetzt.

Alternativ kann die Reaktion in der Flüssigphase bei einem Druck von 40 bis 80 bar abs. und einer Temperatur von 60 bis 120°C in einem Fest-Flüssig-Fließbett- oder einem Strömungsrohrreaktor, der den erfindungsgemäß vorbehandelten Hydroaminierungskatalysator enthält, durchgeführt werden.

Eine besondere Ausführungsform dieses Verfahrens besteht darin, dass man Ammoniak und/oder das primäre bzw. sekundäre Amin zusammen mit dem Olefin oder dem Olefingemisch im molaren Verhältnis 1:1 bis 5:1, bevorzugt 1:1 bis 3:1, gemischt einem Festbettreaktor, der den erfindungsgemäß vorbehandelten Hydroaminierungskatalysator enthält, zuführt und bei einem Druck von 100 bis 320 bar abs., bevorzugt 150 bis 310 bar abs., insbesondere 200 bis 300 bar abs., und einer Temperatur von 200 bis 350°C, bevorzugt 220 bis 330°C, insbesondere 230 bis 320°C, in der Gasphase oder im überkritischen Zustand umsetzt.

Die Lage des Gleichgewichts und damit der Umsatz zum gewünschten Hydroaminierungsprodukt ist stark vom gewählten Reaktionsdruck abhängig. Hoher Druck begünstigt das Additionsprodukt, doch stellt im Allgemeinen aus technischen und wirtschaftlichen Gründen der Druckbereich bis 300 bar abs. das Optimum dar. Die Selektivität der Reaktion wird - neben Größen wie Ammoniak-/Amin-Überschuss und Katalysator - in hohem Maß durch die Temperatur beeinflusst. Zwar nimmt die Reaktionsgeschwindigkeit der Additionsreaktion mit steigender Temperatur stark zu, doch werden gegebenenfalls selektivitätsmindernde Nebenreaktionen gleichzeitig gefördert. Zudem ist eine Temperaturerhöhung aus thermodynamischer Sicht meist nicht vorteilhaft. Die Lage des Temperaturoptimums bezüglich Umsatz und Selektivität ist von der Konstitution des Olefins, des eingesetzten primären Amins und des Katalysators abhängig und liegt meist im Bereich von 220 bis 320°C.

Nach der Umsetzung wird das Produkt der Hydroaminierungsreaktion üblicherweise, z. B. durch Destillation, Rektifikation, Filtration, Wasserwäsche oder Adsorption, getrennt.

Nicht umgesetzte Edukte oder zugeführte Inertgase können in die Reaktion zurückgeführt werden.

Im erfindungsgemäßen Verfahren werden Ammoniak, primäre oder sekundäre Amine eingesetzt. Die primären oder sekundären Amine weisen dabei vorzugsweise C₁₋₂₀-Alkylreste, besonders bevorzugt C₁₋₆-Alkylreste, insbesondere Methylreste oder Ethylreste auf.

Als Olefine können vorzugsweise C₂₋₂₀-Olefine eingesetzt werden, die aliphatisch sind. Sie können dabei linear oder verzweigt sein. Vorzugsweise werden C₂₋₁₂-Olefine, insbesondere C₂₋₆-Olefine eingesetzt. Beispiele geeigneter Olefine sind Ethen, Propen, Buten, Isobuten wie auch 1,3-Butadien. In einer besonders bevorzugten Ausführungsform wird als Olefin iso-Buten eingesetzt.

Neben Ammoniak sind ganz besonders bevorzugte Amine Monomethylamin, Dimethylamin, Monoethylamin, Diethylamin, n-Butylamin, Isopropylamin, Diisopropylamin und Di-n-butylamin. In einer besonders bevorzugten Ausführungsform wird als Ammoniak eingesetzt.

Hydroaminierungsprodukte ausgehend von Ethen und Ammoniak sind Mono-, Di-und/oder Triethylamin, ausgehend von Ethen und Monoethylamin: Di- und/oder Triethylamin, ausgehend von Isobuten und Ammoniak: tert.-Butylamin, ausgehend von 1,3-Butadien und Ammoniak: 1-Amino-3-buten und/oder 2-Amino-3-buten, ausgehend von 1,3-Butadien und n-Butylamin: (2-Butenyl)-n-butylamin und/oder (3-Butenyl)-n-butylamin und ausgehend von Propen und Isopropylamin: Diisopropylamin.

In einer besonders bevorzugten Ausführungsform wird ist das Hydroaminierungsprodukt tert.-Butylamin, ausgehend von iso-Buten und Ammoniak.

Das erfindungsgemäß hergestellte tert. Butylamin kann als Rohstoff in der Gummiindustrie (Vulkanisationsbeschleuniger) oder zur Herstellung von Pflanzenschutzmitteln oder Pharmazeutika verwendet werden.

Durch das erfindungsgemäße Verfahren zur Vorbehandlung von Hydroaminierungskatalysatoren wird in der Regel die thermische Schädigung der Hydroaminierungskatalysatoren beim Anfahren des Katalysators verringert. Somit wird ein Hydroaminierungsverfahren bereitgestellt werden, dass lange Standzeiten aufweist, und dass es ermöglicht Hydroaminierungsprodukt in einer hohen Ausbeute und Selektivität zu erhalten. Die Betriebsdauer des Katalysators bis zu einer ggf. erforderlichen Regenerierung wird durch das erfindungsgemäße Verfahren erhöht. Eine weitere Aufgabe der vorliegenden Erfindung bestand darin, ein Verfahren zum Anfahren eines Hydroaminierungskatalysator zu entwickeln, dass zu einer geringeren thermischen Schädigung der eingesetzten Katalysatoren führt, so dass die Aktivität der Katalysatoren möglichst lange erhalten bleibt.

Die Erfindung wird durch die nachstehenden Beispiele näher erläutert.

### Vergleichsbeispiel 1:

In einem adiabaten Kalorimeter wird ein Hydroamierungskatalysator bestehend aus Zeolith vom Typ SSZ-26 mit Ammoniak und Isobuten beaufschlagt. Die Temperatur zu Beginn der Reaktion (Starttemperatur) beträgt 22°C. Der Temperaturanstieg nach Zugabe der Edukte wird gemessen. Die Temperatur im Kalorimeter steigt auf eine Maximaltemperatur von 75°C. Die genauen Versuchsparameter sind in Tabelle 1 aufgeführt.

### Beispiel 1:

In einem adiabaten Kalorimeter wird ein Hydroamierungskatalysator bestehend aus Zeolith vom Typ SSZ-26 erfindungsgemäß zunächst mit Ammoniak beaufschlagt. Dabei steigt die Temperatur von 21 °C (Starttemperatur) auf 42°C. Nach Entspannung und dadurch Entfernung des Ammoniaks aus dem Kalorimeter wird der Katalysator wieder auf die Starttemperatur temperiert und mit Ammoniak und Isobuten beaufschlagt. Der Temperaturanstieg nach Zugabe der Edukte wird gemessen. Die Temperatur im Kalorimeter steigt auf eine Maximaltemperatur von 24°C. Die genauen Versuchsparameter sind in Tabelle 2 aufgeführt.

**Tabelle 1:**

| | Vergleichsbeispiel | Beispiel 1 |
|---|---|---|
| Volumen Reaktionsgefäß | 114 ml | 114 ml |
| Menge an Katalysator | 33,22 g | 33,83 g |
| Menge an NH₃ zur Vorbehandlung | 0 g | 31,90 g |
| Menge an NH₃/Isobuten-Mischung | 29,17 g | 32,47 g |
| Isobuten-Gehalt der NH₃/Isobuten-Mischung | 69,8 Gew.% | 68,4 Gew. % |
| Starttemperatur | 22°C | 21°C |
| Temperatur nach Zugabe NH₃/Isobuten-Mischung | 75°C | 24°C |

Im großtechnischen Maßstab kann die Wärme, die beim Anfahren bzw. der Beaufschlagung des Katalysators mit den Edukten entsteht, schlecht abgeführt werden, so dass der resultierende Temperaturanstieg zur einer thermischen Schädigung des Katalysators führt. Mit dem erfindungsgemäßen Versuch kann gezeigt werden, dass durch die erfindungsgemäße Vorbehandlung der Hydroaminierungskatalysatoren die bei dem Anfahren von Katalysatoren entstehende Wärmemenge deutlich verringert werden kann, so dass es eine thermische Schädigung des Hydroaminierungskatalysators weitestgehend vermieden wird.

### Vergleichsbeispiel 2:

In einem adiabaten Kalorimeter wird ein Hydroamierungskatalysator bestehend aus Zeolith vom Typ NU-85 mit Ammoniak und Isobuten beaufschlagt. Die Temperatur zu Beginn der Reaktion (Starttemperatur) beträgt 22°C. Der Temperaturanstieg nach Zugabe der Edukte wird gemessen. Die Temperatur im Kalorimeter steigt auf eine Maximaltemperatur von 70°C. Die genauen Versuchsparameter sind in Tabelle 1 aufgeführt.

### Beispiel 2:

In einem adiabaten Kalorimeter wird ein Hydroamierungskatalysator bestehend aus Zeolith vom Typ NU-85 erfindungsgemäß zunächst mit Ammoniak beaufschlagt. Dabei steigt die Temperatur von 21 °C (Starttemperatur) auf 44°C. Nach Entspannung und dadurch Entfernung des Ammoniaks aus dem Kalorimeter wird der Katalysator wieder auf die Starttemperatur temperiert und mit Ammoniak und Isobuten beaufschlagt. Der Temperaturanstieg nach Zugabe der Edukte wird gemessen. Die Temperatur im Kalorimeter steigt auf eine Maximaltemperatur von 25°C. Die genauen Versuchsparameter sind in Tabelle 2 aufgeführt.

**Tabelle 2:**

| | Vergleichsbeispiel | Beispiel 2 |
|---|---|---|
| Volumen Reaktionsgefäß | 114 ml | 114 ml |
| Menge an Katalysator | 33,44 g | 33,25g |
| Menge an NH₃ zur Vorbehandlung | 0 g | 32,05 g |
| Menge an NH₃/Isobuten-Mischung | 30,14 g | 32,37 g |
| Isobuten-Gehalt der NH₃/Isobuten-Mischung | 69,8 Gew.% | 68,4 Gew.% |
| Starttemperatur | 22°C | 21°C |
| Temperatur nach Zugabe NH₃/Isobuten-Mischung | 70°C | 25°C |

### Vergleichsbeispiel 3:

In einem adiabaten Kalorimeter wird ein Hydroamierungskatalysator bestehend aus Zeolith vom Typ MCM-49 mit Ammoniak und Isobuten beaufschlagt. Die Temperatur zu Beginn der Reaktion (Starttemperatur) beträgt 22°C. Der Temperaturanstieg nach Zugabe der Edukte wird gemessen. Die Temperatur im Kalorimeter steigt auf eine Maximaltemperatur von 72°C. Die genauen Versuchsparameter sind in Tabelle 1 aufgeführt.

### Beispiel 3:

In einem adiabaten Kalorimeter wird ein Hydroamierungskatalysator bestehend aus Zeolith vom Typ MCM-49 erfindungsgemäß zunächst mit Ammoniak beaufschlagt. Dabei steigt die Temperatur von 21 °C (Starttemperatur) auf 43°C. Nach Entspannung und dadurch Entfernung des Ammoniaks aus dem Kalorimeter wird der Katalysator wieder auf die Starttemperatur temperiert und mit Ammoniak und Isobuten beaufschlagt. Der Temperaturanstieg nach Zugabe der Edukte wird gemessen. Die Temperatur im Kalorimeter steigt auf eine Maximaltemperatur von 26°C. Die genauen Versuchsparameter sind in Tabelle 2 aufgeführt.

**Tabelle 3:**

| | Vergleichsbeispiel | Beispiel 3 |
|---|---|---|
| Volumen Reaktionsgefäß | 114 ml | 114 ml |
| Menge an Katalysator | 33,75 g | 34,05g |
| Menge an NH₃ zur Vorbehandlung | 0 g | 31,75g |
| Menge an NH₃/Isobuten-Mischung | 31,82 g | 32,57 g |
| Isobuten-Gehalt der NH₃/Isobuten-Mischung | 69,8 Gew.% | 68,4 Gew.% |
| Starttemperatur | 22°C | 21°C |
| Temperatur nach Zugabe NH₃/Isobuten-Mischung | 72°C | 26°C |

### Vergleichsbeispiel 4:

In einem adiabaten Kalorimeter wird ein Hydroamierungskatalysator bestehend aus Bor-BETA-Zeolith in einer γ-Al₂O₃-Matrix (Zeolith-Anteil: 75 Gew.-%) mit Ammoniak und Isobuten beaufschlagt. Die Temperatur zu Beginn der Reaktion (Starttemperatur) beträgt 22°C. Der Temperaturanstieg nach Zugabe der Edukte wird gemessen. Die Temperatur im Kalorimeter steigt auf eine Maximaltemperatur von 73°C. Die genauen Versuchsparameter sind in Tabelle 1 aufgeführt.

### Beispiel 4:

In einem adiabaten Kalorimeter wird ein Hydroamierungskatalysator bestehend aus Bor-BETA-Zeolith in einer γ-Al₂O₃-Matrix (Zeolith-Anteil: 75 Gew.-%) erfindungsgemäß zunächst mit Ammoniak beaufschlagt. Dabei steigt die Temperatur von 21 °C (Starttemperatur) auf 44°C. Nach Entspannung und dadurch Entfernung des Ammoniaks aus dem Kalorimeter wird der Katalysator wieder auf die Starttemperatur temperiert und mit Ammoniak und Isobuten beaufschlagt. Der Temperaturanstieg nach Zugabe der Edukte wird gemessen. Die Temperatur im Kalorimeter steigt auf eine Maximaltemperatur von 26°C. Die genauen Versuchsparameter sind in Tabelle 1 aufgeführt.

**Tabelle 4:**

| | Vergleichsbeispiel | Beispiel 4 |
|---|---|---|
| Volumen Reaktionsgefäß | 114 ml | 114 ml |
| Menge an Katalysator | 33,53g | 33,62g |
| Menge an NH₃ zur Vorbehandlung | 0 g | 31,90 g |
| Menge an NH₃/Isobuten-Mischung | 30,78 g | 31,95 g |
| Isobuten-Gehalt der NH₃/Isobuten-Mischung | 69,8 Gew.% | 68,4 Gew.% |
| Starttemperatur | 22°C | 21°C |
| Temperatur nach Zugabe NH₃/Isobuten-Mischung | 73°C | 26°C |

### Vergleichsbeispiel 5:

In einem adiabaten Kalorimeter wird ein Hydroamierungskatalysator bestehend aus Zeolith vom Typ Bor-MCM-22 mit Ammoniak und Isobuten beaufschlagt. Die Temperatur zu Beginn der Reaktion (Starttemperatur) beträgt 21 °C. Der Temperaturanstieg nach Zugabe der Edukte wird gemessen. Die Temperatur im Kalorimeter steigt auf eine Maximaltemperatur von 78°C. Die genauen Versuchsparameter sind in Tabelle 1 aufgeführt.

### Beispiel 5:

In einem adiabaten Kalorimeter wird ein Hydroamierungskatalysator bestehend aus Zeolith vom Typ Bor-MCM-22 erfindungsgemäß zunächst mit Ammoniak beaufschlagt. Dabei steigt die Temperatur von 22°C (Starttemperatur) auf 44°C. Nach Entspannung und dadurch Entfernung des Ammoniaks aus dem Kalorimeter wird der Katalysator wieder auf die Starttemperatur temperiert und mit Ammoniak und Isobuten beaufschlagt. Der Temperaturanstieg nach Zugabe der Edukte wird gemessen. Die Temperatur im Kalorimeter steigt auf eine Maximaltemperatur von 25°C. Die genauen Versuchsparameter sind in Tabelle 2 aufgeführt.

**Tabelle 5:**

| | Vergleichsbeispiel | Beispiel 5 |
|---|---|---|
| Volumen Reaktionsgefäß | 114 ml | 114 ml |
| Menge an Katalysator | 33,31g | 33,72g |
| Menge an NH₃ zur Vorbehandlung | 0 g | 31,90 g |
| Menge an NH₃/Isobuten-Mischung | 31,15 g | 32,42 g |
| Isobuten-Gehalt der NH₃/Isobuten-Mischung | 69,8 Gew.% | 68,4 Gew.% |
| Starttemperatur | 21°C | 21°C |
| Temperatur nach Zugabe NH₃/Isobuten-Mischung | 78°C | 25°C |

### Vergleichsbeispiel 6:

In einem adiabaten Kalorimeter wird ein Hydroamierungskatalysator bestehend aus Zeolith vom Typ NES mit Ammoniak und Isobuten beaufschlagt. Die Temperatur zu Beginn der Reaktion (Starttemperatur) beträgt 22°C. Der Temperaturanstieg nach Zugabe der Edukte wird gemessen. Die Temperatur im Kalorimeter steigt auf eine Maximaltemperatur von 72°C. Die genauen Versuchsparameter sind in Tabelle 1 aufgeführt.

### Beispiel 6:

In einem adiabaten Kalorimeter wird ein Hydroamierungskatalysator bestehend aus Zeolith vom Typ NES erfindungsgemäß zunächst mit Ammoniak beaufschlagt. Dabei steigt die Temperatur von 22°C (Starttemperatur) auf 42°C. Nach Entspannung und dadurch Entfernung des Ammoniaks aus dem Kalorimeter wird der Katalysator wieder auf die Starttemperatur temperiert und mit Ammoniak und Isobuten beaufschlagt. Der Temperaturanstieg nach Zugabe der Edukte wird gemessen. Die Temperatur im Kalorimeter steigt auf eine Maximaltemperatur von 24°C. Die genauen Versuchsparameter sind in Tabelle 2 aufgeführt.

**Tabelle 6:**

| | Vergleichsbeispiel | Beispiel 6 |
|---|---|---|
| Volumen Reaktionsgefäß | 114 ml | 114 ml |
| Menge an Katalysator | 33,00g | 33,98 g |
| Menge an NH₃ zur Vorbehandlung | 0 g | 31,90 g |
| Menge an NH₃/Isobuten-Mischung | 29,96 g | 29,90 g |
| Isobuten-Gehalt der NH₃/Isobuten-Mischung | 69,8 Gew.% | 68,4 Gew.% |
| Starttemperatur | 22°C | 21°C |
| Temperatur nach Zugabe NH₃/Isobuten-Mischung | 72°C | 24°C |

## Patentansprüche

1. Verfahren zur Vorbehandlung von Hydroaminierungskatalysatoren, **dadurch gekennzeichnet, dass** man den Hydroaminierungskatalysator vor der Umsetzung von Olefinen mit Ammoniak, einem primären oder sekundären Amin mit einem Ammoniak-haltigen Gemisch in Kontakt bringt, wobei das Ammoniak-haltige Gemisch weniger als 40 Gew.-% Olefin enthält.

2. Verfahren nach Anspruch 1, **dadurch gekennzeichnet, dass** das Ammoniak-haltige Gemisch weniger als 10 Gew.% Olefin enthält.

3. Verfahren nach mindestens einem der Ansprüche 1 bis 2, **dadurch gekennzeichnet, dass** man das Ammoniak-haltige Gemisch zusammen mit einem oder mehreren Inertgasen mit den Hydroaminierungskatalysator in Kontakt bringt.

4. Verfahren nach mindestens einem der Ansprüche 1 bis 3, **dadurch gekennzeichnet, dass** man den Hydroaminierungskatalysator mit dem Ammoniak-haltigen Gemisch bei einem Druck von 0,1 bis 50 bar abs. in Kontakt bringt.

5. Verfahren nach mindestens einem der Ansprüche 1 bis 4, **dadurch gekennzeichnet, dass** man den Hydroaminierungskatalysator mit dem Ammoniak-haltige Gemisch bei einer Temperatur von 0 bis 50°C in Kontakt bringt.

6. Verfahren nach mindestens einem der Ansprüche 1 bis 5, **dadurch gekennzeichnet, dass** man einen Hydroaminierungskatalysator einsetzt, der zuvor noch nicht in eine Hydroaminierungsreaktion eingesetzt worden ist.

7. Verfahren nach mindestens einem der Ansprüche 1 bis 6, **dadurch gekennzeichnet, dass** man einen regenerierten Hydroaminierungskatalysator einsetzt.

8. Verfahren nach mindestens einem der Ansprüche 1 bis 7, **dadurch gekennzeichnet, dass** man einen thermisch aktivierten Hydroaminierungskatalysator einsetzt.

9. Verfahren nach mindestens einem der Ansprüche 1 bis 8, **dadurch gekennzeichnet, dass** man einen calcinierten zeolithischen Hydroaminierungskatalysator einsetzt.

10. Verfahren nach mindestens einem der Ansprüche 1 bis 9, **dadurch gekennzeichnet, dass** die Vorbehandlung in einem kontinuierlich betriebenen Reaktor erfolgt.

11. Verfahren nach mindestens einem der Ansprüche 1 bis 10, **dadurch gekennzeichnet, dass** die Vorbehandlung in einem adiabat betrieben Reaktor erfolgt.

12. Verfahren zur Herstellung von Alkylaminen durch Umsetzung von Olefinen mit Ammoniak, primären oder sekundären Aminen an einem Hydroaminierungskatalysator, **dadurch gekennzeichnet, dass** man vor der Umsetzung der Olefine mit Ammoniak, primären oder sekundären Aminen den Hydroaminierungskatalysator gemäß mindestens einem der Ansprüche 1 bis 11 vorbehandelt.

13. Verfahren nach Anspruch 12, **dadurch gekennzeichnet, dass** das molare Verhältnis von Ammoniak, primären oder sekundären Aminen zu Olefin zwischen 1:1 und 3:1 beträgt.

14. Verfahren nach mindestens einem der Ansprüche 12 bis 13, **dadurch gekennzeichnet, dass** Olefine mit Ammoniak umgesetzt werden.

15. Verfahren nach mindestens einem der Ansprüche 12 bis 14, **dadurch gekennzeichnet, dass** man als Olefin iso-Buten einsetzt.

16. Verfahren zur Herstellung von Pflanzenschutzmitteln oder Pharmazeutika oder Gummi, **dadurch gekennzeichnet, dass** man in einer ersten Stufe tertiär-Butylamin gemäß Anspruch 13 herstellt und in einer zweiten Stufe das in der ersten Stufe erhaltene tertiär-Butylamin in ein Verfahren zur Herstellung von Pflanzenschutzmitteln oder Pharmazeutika oder Gummi einsetzt.

## Claims

1. A process for the pretreatment of hydroamination catalysts, wherein the hydroamination catalyst is brought into contact with an ammonia-comprising mixture before the reaction of olefins with ammonia, a primary or secondary amine, with the ammonia-comprising mixture comprising less than 40% by weight of olefin.

2. The process according to claim 1, wherein the ammonia-comprising mixture comprises less than 10% by weight of olefin.

3. The process according to at least one of claims 1 and 2, wherein the ammonia-comprising mixture is brought into contact with the hydroamination catalyst together with one or more inert gases.

4. The process according to at least one of claims 1 to 3, wherein the hydroamination catalyst is brought into contact with the ammonia-comprising mixture at a pressure of from 0.1 to 50 bar abs.

5. The process according to at least one of claims 1 to 4, wherein the hydroamination catalyst is brought into contact with the ammonia-comprising mixture at a temperature of from 0 to 50°C.

6. The process according to at least one of claims 1 to 5, wherein a hydroamination catalyst which has not previously been used in a hydroamination reaction is used.

7. The process according to at least one of claims 1 to 6, wherein a regenerated hydroamination catalyst is used.

8. The process according to at least one of claims 1 to 7, wherein a thermally activated hydroamination catalyst is used.

9. The process according to at least one of claims 1 to 8, wherein a calcined zeolytic hydroamination catalyst is used.

10. The process according to at least one of claims 1 to 9, wherein the pretreatment is carried out in a continuously operated reactor.

11. The process according to at least one of claims 1 to 10, wherein the pretreatment is carried out in an adiabatically operated reactor.

12. A process for preparing alkylamines by reaction of olefins with ammonia, primary or secondary amines over a hydroamination catalyst, wherein the hydroamination catalyst is pretreated according to at least one of claims 1 to 11 before the reaction of the olefins with ammonia, primary or secondary amines.

13. The process according to claim 12, wherein the molar ratio of ammonia, primary or secondary amines to olefin is in the range from 1:1 to 3:1.

14. The process according to at least one of claims 12 and 13, wherein olefins are reacted with ammonia.

15. The process according to at least one of claims 12 to 14, wherein isobutene is used as olefin.

16. A process for preparing crop protection agents or pharmaceuticals or rubber, wherein tert-butylamine is prepared according to claim 13 in a first stage and the tert-butylamine obtained in the first stage is used in a process for preparing crop protection agents or pharmaceuticals or rubber in a second stage.

## Revendications

1. Procédé pour le prétraitement de catalyseurs d'hydroamination, **caractérisé en ce qu'**on met en contact le catalyseur d'hydroamination, avant la transformation d'oléfines avec de l'ammoniac, une amine primaire ou une amine secondaire, avec un mélange contenant de l'ammoniac, le mélange contenant de l'ammoniac contenant moins de 40% en poids d'oléfine.

2. Procédé selon la revendication 1, **caractérisé en ce que** le mélange contenant de l'ammoniac contient moins de 10% en poids d'oléfine.

3. Procédé selon au moins l'une quelconque des revendications 1 à 2, **caractérisé en ce qu'**on met en contact le mélange contenant de l'ammoniac, ensemble avec un ou plusieurs gaz inertes, avec le catalyseur d'hydroamination.

4. Procédé selon au moins l'une quelconque des revendications 1 à 3, **caractérisé en ce qu'**on met en contact le catalyseur d'hydroamination avec le mélange contenant de l'ammoniac à une pression de 0,1 à 50 bars abs.

5. Procédé selon au moins l'une quelconque des revendications 1 à 4, **caractérisé en ce qu'**on met en contact le catalyseur d'hydroamination avec le mélange contenant de l'ammoniac à une température de 0 à 50°C.

6. Procédé selon au moins l'une quelconque des revendications 1 à 5, **caractérisé en ce qu'**on utilise un catalyseur d'hydroamination qui n'a pas encore été utilisé au préalable dans une réaction d'hydroamination.

7. Procédé selon au moins l'une quelconque des revendications 1 à 6, **caractérisé en ce qu'**on utilise un catalyseur d'hydroamination régénéré.

8. Procédé selon au moins l'une quelconque des revendications 1 à 7, **caractérisé en ce qu'**on utilise un catalyseur d'hydroamination thermiquement activé.

9. Procédé selon au moins l'une quelconque des revendications 1 à 8, **caractérisé en ce qu'**on utilise un catalyseur d'hydroamination zéolithique calciné.

10. Procédé selon au moins l'une quelconque des revendications 1 à 9, **caractérisé en ce que** le prétraitement a lieu dans un réacteur exploité en continu.

11. Procédé selon au moins l'une quelconque des revendications 1 à 10, **caractérisé en ce que** le prétraitement a lieu dans un réacteur exploité de manière adiabatique.

12. Procédé pour la préparation d'alkylamines par transformation d'oléfines avec de l'ammoniac, des amines primaires ou secondaires sur un catalyseur d'hydroamination, **caractérisé en ce qu'**on prétraite, avant la transformation des oléfines avec de l'ammoniac, des amines primaires ou des amines secondaires, le catalyseur d'hydroamination selon au moins l'une quelconque des revendications 1 à 11.

13. Procédé selon la revendication 12, **caractérisé en ce que** le rapport molaire de l'ammoniac, des amines primaires ou des amines secondaires à l'oléfine vaut entre 1:1 et 3:1.

14. Procédé selon au moins l'une quelconque des revendications 12 à 13, **caractérisé en ce que** les oléfines sont transformées avec de l'ammoniac.

15. Procédé selon au moins l'une quelconque des revendications 12 à 14, **caractérisé en ce qu'**on utilise, comme oléfine, de l'isobutène.

16. Procédé pour la préparation d'agents de phytoprotection ou de produits pharmaceutiques ou de caoutchouc, **caractérisé en ce qu'**on prépare, dans une première étape, de la tert-butylamine selon la revendication 13 et on utilise dans une deuxième étape la tert-butylamine obtenue dans la première étape dans un procédé de préparation d'agents de phytoprotection ou de produits pharmaceutiques ou de caoutchouc.
